# EUROPEAN PATENT APPLICATION

(11) **EP 3 721 906 A1**
(43) Date of publication of application: **14.10.2020**
(21) Application number: 18885829.4
(22) Date of filing: 05.12.2018
(51) Int. Cl.: A61K 45/06, A61K 31/444, A61K 31/497, A61P 35/04

(54) **USE OF PARP INHIBITOR IN TREATING CHEMOTHERAPY-RESISTANT OVARIAN CANCER OR BREAST CANCER**

(30) Priority: 06.12.2017 CN 201711278350
(71) Applicant: Jiangsu Hengrui Medicine Co., Ltd., Lianyungang, Jiangsu 222047 (CN)
(72) Inventor: WANG, Quanren, Lianyungang Jiangsu 222047 (CN)
(74) Representative: Potter Clarkson
(86) International application number: PCT/CN2018/119318
(87) International publication number: WO 2019/109938

(57) **Abstract**

The present invention provides a use of a PARP inhibitor in treating a chemotherapy-resistant ovarian cancer or breast cancer. In particular, the present invention provides a use of a PARP inhibitor in preparing a drug for treating a chemotherapy-resistant ovarian cancer or breast cancer. The PARP inhibitor is selected from a compound as shown in general formula (B) and a pharmaceutically acceptable salt thereof.

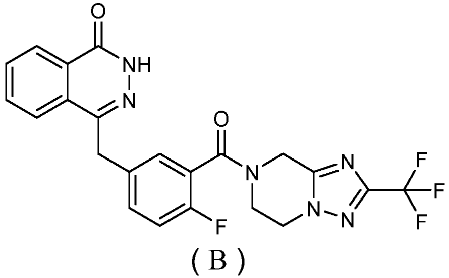

## Description

### FIELD OF THE INVENTION

The present invention relates to a use of a PARP inhibitor in the preparation of a medicament for treating chemotherapy-resistant ovarian cancer or breast cancer.

### BACKGROUND OF THE INVENTION

Epithelial ovarian cancer (EOC) is the most lethal gynecological malignant tumor. Nearly 75% of patients are in the advanced stage at the time oftreatment, and require a radical surgery and a standard treatment regimen with platinum-based chemotherapy. Although the complete response rate after standard treatment can reach 40% to 60%, more than 90% of patients will relapse in an average of 18 months, and then face the risk of death due to chemotherapy resistance and disease progression.

With respect to recurrent ovarian cancer, if the patient is sensitive to the platinum-based chemotherapy regimen (the time from the last chemotherapy to tumor recurrence/progression ≥ 6 months), the platinum-based treatment regimen will continue to be used in follow-up treatment. If the patient is resistant to the platinum-based chemotherapy regimen (the time from the last chemotherapy to tumor recurrence/progression < 6 months), a single-agent chemotherapy will be used in follow-up treatment. Chemotherapy regimen includes docetaxel, paclitaxel, liposomal doxorubicin, gemcitabine and the like. The effect of these treatments is limited. The tumor response rate during treatment is generally 15 to 20%, and the progression-free survival period is about 3 to 4 months. There is an urgent need to develop a high-efficiency and low-toxicity treatment regimen for recurrent ovarian cancer.

Breast cancer is a type of disease that is highly heterogeneous in morphology, molecular biology, clinical manifestation and response to treatment. Triple negative breast cancer (TNBC) is a special subtype, accounting for 12% to 17% of all breast cancers. It appears negative for estrogen receptor (ER), progesterone receptor (PR) and human epidermal growth factor receptor 2 (HER2). This type of breast cancer has a higher incidence in African Americans, young women before menopause, and patients with BRCA1 mutation. It has a poor cell differentiation, high invasiveness, high risk of distant metastasis, and more tendency to visceral metastasis. Studies have shown that the median survival time of recurrent and metastatic TNBC is only 13 months, and the 5-year survival rate is less than 30%. Since triple negative breast cancer lacks targets for endocrine therapy and molecular targeted therapy, chemotherapy is the main systemic treatment for triple negative breast cancer patients.

Drug resistance is a difficult problem in the treatment of ovarian cancer and breast cancer, which can generally be divided into congenital drug resistance (also called primary drug resistance) and secondary drug resistance (also called recurrent drug resistance). After the recurrence of ovarian cancer, determining whether the patient is platinum-based compound-sensitive or platinum-based compound-resistant is important for the establishment of treatment regimen.

Poly(adenosine diphosphate-ribose) polymerase or poly (ADP-ribose) polymerase (PARP) plays an important role in repairing DNA single-strand break (SSB) induced by various causes. Since the PARP inhibitor Olaparib was approved in 2014 for treating ovarian cancer with BRCA1/2 mutation, the development of PARP inhibitors for anti-tumor treatment has developed rapidly. Preclinical studies have demonstrated that in addition to being applied as a single drug, PARP inhibitors can also be used as a radiosensitizer or chemosensitizer in combination with radiotherapy or chemotherapy, so as to enhance anti-tumor efficacy and reduce the drug dose in chemotherapy or radiation dose in the radiotherapy, and reduce toxic and side effects. A recent phase II clinical trial showed that Olaparib showed an 88% response to advanced prostate cancer patients with DNA repair gene mutations, which allowed tumor growth to be inhibited or even reduced, and the overall survival was longer than the expected survival of the same type patients. Therefore, the continuous expansion of the use mode and indication scope of PARP inhibitors has a good promotion for the development and application of PARP inhibitors. WO2012019427A1 (publication date of February 16, 2012) discloses a PARP inhibitor capable of inhibiting the growth of various tumors, and its structure is shown in formula (B)

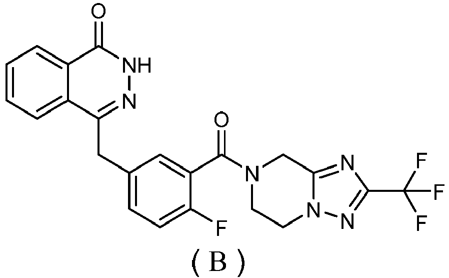

Vascular endothelial growth factor (VEGF) is the most important positive regulatory protein ever identified. VEGF induces phosphorylation of its receptor subtype VEGFR-2 by binding to it, and further activates a series of cascades that cause vascular endothelial cell proliferation and induce angiogenesis. Studies have shown that VEGF and its receptors are highly expressed in gastric cancer tissues, and their expression levels are positively correlated with the prognosis of gastric cancer. Therefore, the treatment of targeting VEGF or its receptors to destroy neovascularization will undoubtedly provide a novel therapeutic direction and molecular target for patients with gastric cancer. Bevacizumab is a recombinant human anti-VEGF monoclonal antibody, and is the first drug approved for anti-angiogenesis of tumor. WO2005000232A2 (publication date of January 6, 2005) discloses a small molecule tyrosine kinase inhibitor Apatinib, which highly selectively competes for the ATP-binding site of VEGFR-2 in cells, blocks the downstream signal transduction, inhibits the neovascularization of tumor, and finally achieves the purpose of treating tumors. The structure of Apatinib is shown in formula (I)

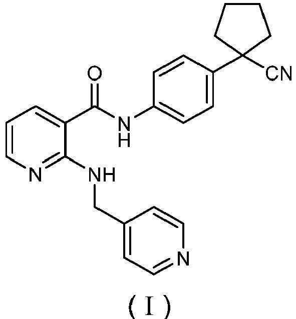

The combination of more than one anti-tumor drugs that have different targets and are interrelated is a generally accepted anti-tumor therapy, which fully utilizes the advantages of each component, and can not only improve the anti-tumor activity of each single drug but also reduce the toxicity of the drugs. A phase II clinical trial of the combination of a PARP inhibitor Olaparib and Cediranib (a drug that inhibits neovascularization by inhibiting VEGFR activity) initially demonstrated that the combination regimen is more effective than the two drugs used alone in patients with recurrent ovarian cancer that are sensitive to the platinum-based compound treatment, indicating that the combination regimen of the above two drugs with different targets has a good feasibility for tumor treatment.

Patent applications WO2010096627A1 (publication date of August 26, 2010), WO2014004376A2 (publication date of January 3, 2014), WO2016116602A1 (publication date of July 28, 2016) and WO2016179123A1 (publication date of November 10, 2016) disclose the use of a combination of a VEGFR inhibitor and a PARP inhibitor in treating malignant tumors (such as breast or ovarian cancer, etc). However, the existing combination regimens are aimed at ovarian cancer that is sensitive to the platinum-based treatment, whether the combination has a synergistic effect on the inhibition of recurrent chemotherapy-resistant ovarian cancer or breast cancer or not is unknown.

### SUMMARY OF THE INVENTION

The technical problem to be solved by the present invention is to provide a use of a poly(adenosine diphosphate-ribose) polymerase (PARP) inhibitor in the preparation of a medicament for treating chemotherapy-resistant ovarian cancer or breast cancer.

The technical solutions of the present invention are as follows:
The present invention provides a use of a PARP inhibitor in the preparation of a medicament for treating chemotherapy-resistant ovarian cancer or chemotherapy-resistant breast cancer.

Preferably, the PARP inhibitor is selected from the group consisting of Olaparib, Talazoparib, Veliparib, Rucaparib, CEP-8983 and BGB-290.

In a preferred embodiment, the PARP inhibitor is a compound of formula (B) or a pharmaceutically acceptable salt thereof,

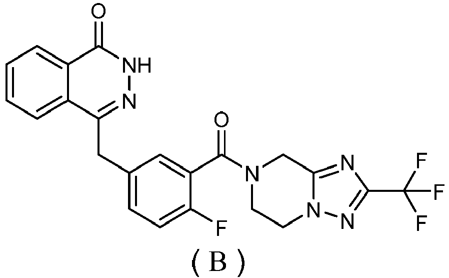

Further, the combination of the poly(adenosine diphosphate-ribose) polymerase inhibitor and the VEGFR inhibitor has a synergistic effect. The VEGFR inhibitor is a VEGFR-2 inhibitor that includes, but is not limited to, PAN-90806, Foretinib, Tafetinib, Kanitinib, Apatinib, Tanibirumab, Anlotinib, Lucitanib, Vatalanib, Cediranib, Chiauranib, Dovitinib, Donafenib, Famitinib, Sitravatinib, Telatinib, L-21649, TAS-115, Cabozantinib, Thiophenib, Fruquintinib, Brivanib, Sulfatinib, Ramucirumab, Glesatinib, Nintedanib, Puquitinib, Axitinib, EDP317, Sorafenib, Metatinib, Tivozanib, Regorafenib, Midostaurin, Pazopanib, HLX-06, Altiratinib, Ningetinib, Sunitinib, AL-8326, Rebastinib and pharmaceutically acceptable salt thereof.

In a preferred embodiment, the VEGFR-2 inhibitor is a compound of formula (I) or a pharmaceutically acceptable salt thereof,

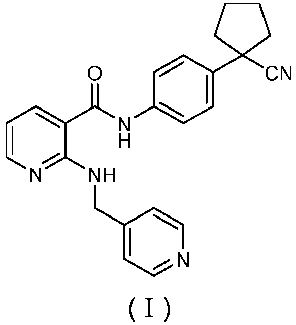

In the above embodiments, the pharmaceutically acceptable salt includes, but is not limited to, hydrochloride, mesylate, maleate, malate and besylate, and preferably mesylate.

The combination of the present invention has a synergistic effect.

In some embodiments, the administration dose of the poly(adenosine diphosphate-ribose) polymerase inhibitor is 0.1 to 1000 mg, and can be 0.1 mg, 0.3 mg, 0.5 mg, 0.7 mg, 0.9 mg, 0 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 495 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, or 1000 mg.

In another optional embodiment, the administration dose of the VEGFR inhibitor is 0.1 to 1000 mg, and can be 0.1 mg, 0.3 mg, 0.5 mg, 0.7 mg, 0.9 mg, 0 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 125 mg, 150 mg, 175 mg, 200 mg, 225 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 825 mg, 850 mg, 875 mg, 900 mg, 925 mg, 950 mg, 975 mg, or 1000 mg.

In a preferred embodiment, the ratio of the administration dose of the VEGFR inhibitor to the administration dose of the poly(adenosine diphosphate-ribose) polymerase inhibitor is 0.001 to 1000.

In an optional embodiment, the ratio of the daily administration dose of the VEGFR inhibitor to the daily administration dose of the poly(adenosine diphosphate-ribose) polymerase inhibitor is 0.5:1 to 2:1, and can be 0.50:1, 0.51:1, 0.52:1, 0.53:1, 0.54:1, 0.55:1, 0.56:1, 0.57:1, 0.58:1, 0.59:1, 0.60:1, 0.61:1, 0.62:1, 0.63:1, 0.64:1, 0.65:1, 0.66:1, 0.67:1, 0.68:1, 0.69:1, 0.70:1, 0.71:1, 0.72:1, 0.73:1, 0.74:1, 0.75:1, 0.76:1, 0.77:1, 0.78:1, 0.79:1, 0.80:1, 0.81:1, 0.82:1, 0.83:1, 0.84:1, 0.85:1, 0.86:1, 0.87:1, 0.88:1, 0.89:1, 0.90:1, 0.91:1, 0.92:1, 0.93:1, 0.94:1, 0.95:1, 0.96:1, 0.97:1, 0.98:1, 0.99:1, 1.00:1, 1.01:1, 1.02:1, 1.03:1, 1.04:1, 1.05:1, 1.06:1, 1.07:1, 1.08:1, 1.09:1, 1.10:1, 1.11:1, 1.12:1, 1.13:1, 1.14:1, 1.15:1, 1.16:1, 1.17:1, 1.18:1, 1.19:1, 1.20:1, 1.21:1, 1.22:1, 1.23:1, 1.24:1, 1.25:1, 1.26:1, 1.27:1, 1.28:1, 1.29:1, 1.30:1, 1.31:1, 1.32:1, 1.33:1, 1.34:1, 1.35:1, 1.36:1, 1.37:1, 1.38:1, 1.39:1, 1.40:1, 1.41:1, 1.42:1, 1.43:1, 1.44:1, 1.45:1, 1.46:1, 1.47:1, 1.48:1, 1.49:1, 1.50:1, 1.51:1, 1.52:1, 1.53:1, 1.54:1, 1.55:1, 1.56:1, 1.57:1, 1.58:1, 1.59:1, 1.60:1, 1.61:1, 1.62:1, 1.63:1, 1.64:1, 1.65:1, 1.66:1, 1.67:1, 1.68:1, 1.69:1, 1.70:1, 1.71:1, 1.72:1, 1.73:1, 1.74:1, 1.75:1, 1.76:1, 1.77:1, 1.78:1, 1.79:1, 1.80:1, 1.81:1, 1.82:1, 1.83:1, 1.84:1, 1.85:1, 1.86:1, 1.87:1, 1.88:1, 1.89:1, 1.90:1, 1.91:1, 1.92:1, 1.93:1, 1.94:1, 1.95:1, 1.96:1, 1.97:1, 1.98:1, 1.99:1, or 2.00:1, and preferably is 0.83:1, 1.25:1, 1.56:1, or 1.88:1.

Further, in an optional embodiment, the administration dose of the poly(adenosine diphosphate-ribose) polymerase inhibitor is 1 to 500 mg, and preferably 2.5 mg, 3 mg, 5 mg, 6 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 495 mg, or 500 mg, and the administration dose of the VEGFR inhibitor is 1 to 850 mg, and preferably 2.5 mg, 3 mg, 5 mg, 6 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 495 mg, 500 mg, 500 mg, 505 mg, 510 mg, 515 mg, 520 mg, 525 mg, 530 mg, 535 mg, 540 mg, 545 mg, 550 mg, 555 mg, 560 mg, 565 mg, 570 mg, 575 mg, 580 mg, 585 mg, 590 mg, 595 mg, 600 mg, 605 mg, 610 mg, 615 mg, 620 mg, 625 mg, 630 mg, 635 mg, 640 mg, 645 mg, 650 mg, 655 mg, 660 mg, 665 mg, 670 mg, 675 mg, 680 mg, 685 mg, 690 mg, 695 mg, 700 mg, 705 mg, 710 mg, 715 mg, 720 mg, 725 mg, 730 mg, 735 mg, 740 mg, 745 mg, 750 mg, 755 mg, 760 mg, 765 mg, 770 mg, 775 mg, 780 mg, 785 mg, 790 mg, 795 mg, 800 mg, 805 mg, 810 mg, 815 mg, 820 mg, 825 mg, 830 mg, 835 mg, 840 mg, 845 mg, or 850 mg, and more preferably 50 mg, 100 mg, 150 mg, 200 mg, 250 mg, 375 mg, 425 mg, 500 mg, 600 mg, 700 mg, 750 mg, 800 mg, or 850 mg.

In a preferred embodiment, the administration dose of the poly(adenosine diphosphate-ribose) polymerase inhibitor is 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg (twice a day), or an escalated dose of 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg (once a day) respectively, in combination with a fixed dose of 250 mg or 375 mg of the VEGFR inhibitor (once a day).

In an optional embodiment, the VEGFR inhibitor is recommended to be administrated once a day, and the poly(adenosine diphosphate-ribose) polymerase inhibitor is recommended to be administrated twice a day with an interval of ∼12 hours.

The chemotherapy-resistance of the present invention comprises primary chemotherapy-resistance and recurrent chemotherapy-resistance, and is preferably recurrent chemotherapy-resi stance.

The recurrent chemotherapy-resistant ovarian cancer or recurrent chemotherapy-resistant breast cancer of the present invention means that the cancer cell group that was originally sensitive to the drug developed resistance during repeated treatments and repeated contact with drugs for treating ovarian cancer or breast cancer. The drug for treating ovarian cancer includes, but is not limited to, at least one of gemcitabine, paclitaxel, bevacizumab, sorafenib, sunitinib, pazopanib, lenvatinib mesylate, carboplatin, taxotere, pemetrexed disodium, everolimus, erlotinib, neratinib, gefitinib, Nintedanib, dasatinib, Trametinib, avelumab, ribociclib, Ipilimumab, Lobaplatin, Enzalutamide, mifepristone, Olaparib, alkylating agent, camphorsulfonic acid, Clovis, imatinib, s-malate 184, azacitidine, cetuximab, Alza, anastrozole, exemestane, copanlisib hydrochloride, irinotecan, lenalidomide, pertuzumab, vismodegib, aflibercept, interleukin, topotecan, docetaxel, levonorgestrel, panitumumab, vinflunine, filgrastim, letrozole, cabazitaxel, doxorubicin, altretamine, dianhydrogalactitol, sirolimus, aminopterin, vandetanib, subsequent derivatives of interferon alpha-2b, pharmbiotek, recombinant human interferon alpha-2b, bendamustine, belinostat, immunocell LC, interferon gamma-lb, catumaxomab, glutoxim, Vorinostat, cetrorelix, ponatinib, treosulfan, etoposide, bexarotene, paclitaxel, lobaplatin, ixabepilone, taxotere, mitoxantrone, mifepristone, paclitaxel, doxorubicin hydrochloride, belotecan, raltitrexed, paclitaxel, alitretinoin, doxorubicin hydrochloride, satumomab, pendetide, topotecan hydrochloride, nedaplatin, docetaxel, cisplatin, oxaliplatin/platinum oxalate, and preferably platinum-based compounds such as carboplatin, cisplatin, oxaliplatin/platinum oxalate, sulfatodiamino cyclohexane platinum, nedaplatin or lobaplatin. The drug for treating breast cancer includes, but is not limited to, at least one of trastuzumab, atezolizumab, gemcitabine, paclitaxel, Pembrolizumab, ramucirumab, durvalumab, bevacizumab, oxaliplatin, capecitabine, Nivolumab, ruxolitinib, tamoxifen, lenvatinib mesylate, paclitaxel, everolimus, neratinib, gefitinib, avelumab, ribociclib, Enzalutamide, palbociclib, alkylating agent, azacitidine, trastuzumab, abiraterone, doxorubicin, abemaciclib, anastrozole, zoledronic acid, eribulin mesylate, pertuzumab, docetaxel, epirubicin, lapatinib, letrozole, cabazitaxel, trastuzumab, vandetanib, axitinib, interferon gamma-lb, trastuzumab biosimilars, romidepsin, bicalutamide, taxotere, uroacitides, amrubicin, ixabepilone, mifepristone, paclitaxel, toremifene, cisplatin, paclitaxel, and preferably gemcitabine, docetaxel, epirubicin, paclitaxel, lapatinib or platinum-based compounds such as carboplatin, cisplatin, oxaliplatin/platinum oxalate, sulfatodiamino cyclohexane platinum, nedaplatin or lobaplatin.

The present invention provides a use of a combination of a VEGFR inhibitor and a poly(adenosine diphosphate-ribose) polymerase inhibitor in the preparation of a medicament for treating platinum-based compound-resistant ovarian cancer or breast cancer.

Preferably, the present invention provides a use of a combination of a VEGFR inhibitor and a poly(adenosine diphosphate-ribose) polymerase inhibitor in the preparation of a medicament for treating recurrent platinum-based compound-resistant ovarian cancer.

Preferably, the present invention provides a use of a combination of a VEGFR inhibitor and a poly(adenosine diphosphate-ribose) polymerase inhibitor in the preparation of a medicament for treating recurrent platinum-based compound-resistant breast cancer.

The ovarian cancer of the present invention is selected from the group consisting of high grade serous ovarian cancer, fallopian tube or primary peritoneal cancer, epithelial ovarian cancer and ovarian tumor.

The breast cancer of the present invention is preferably triple negative breast cancer.

The present invention also provides a use of the compound of formula (B) or a pharmaceutically acceptable salt thereof in the preparation of a medicament for treating previously platinum-based compound-sensitive ovarian cancer, breast cancer failed to previous chemotherapy or chemotherapy-resistant breast cancer.

Further, the poly(adenosine diphosphate-ribose) polymerase inhibitor i.e., the compound of formula (B) or a pharmaceutically acceptable salt thereof is used in combination with a VEGFR inhibitor, the combination has a synergistic effect, and the VEGFR inhibitor is preferably the compound of formula (I) or a pharmaceutically acceptable salt thereof.

The present invention also provides a use of the combination of the compound of formula (B) or a pharmaceutically acceptable salt thereof and the compound of formula (I) or a pharmaceutically acceptable salt thereof in the preparation of a medicament for treating ovarian cancer or breast cancer.

In an optional embodiment, the ovarian cancer is selected from the group consisting of high grade serous ovarian cancer, fallopian tube or primary peritoneal cancer, epithelial ovarian cancer and ovarian tumor.

In an optional embodiment, the ovarian cancer is chemotherapy-resistant, and preferably recurrent chemotherapy-resistant.

In a preferred embodiment, the ovarian cancer is platinum-based compound-resistant, and more preferably recurrent platinum-based compound-resistant.

In a preferred embodiment, the ovarian cancer is platinum-based compound-sensitive, and preferably previously platinum-based compound-sensitive. In an optional embodiment, the breast cancer is triple negative breast cancer.

Further, the poly(adenosine diphosphate-ribose) polymerase inhibitor i.e., the compound of formula (B) or a pharmaceutically acceptable salt thereof is used in combination with a VEGFR inhibitor, and the combination has a synergistic effect.

In some embodiments, the poly(adenosine diphosphate-ribose) polymerase inhibitor is administrated alone, i.e., it does not need to be used in combination with other anti-tumor drugs, but some adjuvant drugs that do not have anti-tumor effect are not excluded.

The present invention also provides a pharmaceutical composition of a poly(adenosine diphosphate-ribose) polymerase inhibitor and a VEGFR inhibitor, comprising optional one or more pharmaceutically acceptable vehicles, excipients and/or diluents. The pharmaceutical composition can be formulated into any one of the pharmaceutically acceptable dosage forms. For example, the pharmaceutical formulation of the poly(adenosine diphosphate-ribose) polymerase inhibitor and the VEGFR inhibitor can be formulated into a tablet, capsule, pill, granule, solution, suspension, syrup, injection (including injection solution, sterile powder for injection and concentrated solution for injection), suppository, inhalant or spray.

In addition, the pharmaceutical composition of the present invention can also be administrated to a patient or subject in need of such treatment by any suitable administration mode, for example, oral, parenteral, rectal, pulmonary or topical administration etc. When administrated orally, the pharmaceutical composition can be formulated into an oral formulation, for example, an oral solid formulation such as a tablet, capsule, pill, granule and the like; or an oral liquid formulation such as an oral solution, oral suspension, syrup and the like. When formulated into an oral formulation, the pharmaceutical formulation can also comprise a suitable filler, binder, disintegrant, lubricant and the like.

The pharmaceutical composition of the poly(adenosine diphosphate-ribose) polymerase inhibitor and the VEGFR inhibitor of the present invention can be administrated alone, or in combination with one or more therapeutic agents. Therefore, in certain preferred embodiments, the pharmaceutical composition also comprises one or more therapeutic agents. In certain preferred embodiments, the therapeutic agent is selected from the group consisting of: an antibody, alkylating agent, antimetabolite, antibiotic, alkaloid and hormone, wherein the alkylating agent is selected from the group consisting of bendamustine and temozolomide, the antimetabolite is selected from the group consisting of 5-fluorouracil and cytarabine, the antibody is herceptin, the antibiotic is selected from the group consisting of adriamycin and mitomycin C, the alkaloid is selected from the group consisting of vinblastine and harringtonine, and the hormone is selected from the group consisting of prednisone and thyroxine.

The components to be combined (for example, the VEGFR inhibitor and the poly(adenosine diphosphate-ribose) polymerase inhibitor as well as the second therapeutic agent) can be administrated simultaneously or sequentially separately. For example, the second therapeutic agent can be administrated before, at the same time of, or after the co-administration of the VEGFR inhibitor and the poly(adenosine diphosphate-ribose) polymerase inhibitor of the present invention. Moreover, the components to be combined can also be co-administrated in the same formulation or in different formulations separately.

The present invention also provides a method for treating recurrent chemotherapy-resistant ovarian cancer, comprising administrating to a cancer patient the aforementioned poly(adenosine diphosphate-ribose) polymerase inhibitor and the aforementioned VEGFR inhibitor.

Further, the method comprises a step of determining whether the patient is platinum-based compound-sensitive or platinum-based compound-resistant after the recurrence of ovarian cancer.

The present invention also provides a method for treating ovarian cancer, comprising administrating to a cancer patient the aforementioned compound of formula (B) or a pharmaceutically acceptable salt thereof and the aforementioned compound of formula (I) or a pharmaceutically acceptable salt thereof.

Further, the method comprises a step of determining whether the patient is platinum-based compound-sensitive or platinum-based compound-resistant after the recurrence of ovarian cancer.

The present invention also provides a method for treating breast cancer, comprising administrating to a cancer patient the aforementioned poly(adenosine diphosphate-ribose) polymerase inhibitor and the aforementioned VEGFR inhibitor. If not explained to the contrary, the terms in the present invention have the following meanings:
According to the present invention, the patient subjected to a recurrence within 6 months after the end of platinum-based chemotherapy regimen is platinum-resistant; while the patient subjected to a recurrence more than 6 months after the end of chemotherapy is platinum-based compound-sensitive (also called platinum-treatment-sensitive).

The recurrent drug resistance of the present invention is also called secondary drug resistance.

The enrolled subject of the present invention is preferably a patient with recurrent ovarian cancer (it should be epithelial ovarian cancer, fallopian tube cancer or primary peritoneal cancer that is high-grade serous and/or known to have BRCA1/2 dysfunctional mutation) diagnosed by histology or cytology, who had received 2 to 4 times of platinum-based regimen treatment, the efficacy was non-PD during the last platinum-based regimen treatment, and the cancer was relapsed/progressed <6 months after the end of the treatment; or who was intolerant to the toxicity during the treatment, and the cancer was relapsed/progressed ≥6 months after the end of the penultimate platinum-based regimen treatment; or a patient with recurrent and metastatic triple negative breast cancer diagnosed by histology or cytology, who had been subjected to no more than two times of chemotherapy treatment after recurrence and metastasis and received treatment failure.

Note: toxicity intolerance: hematological toxicity of grade 4 or non-hematological toxicity of grade 3 and above occurs during the treatment. The definition of treatment failure: the disease is progressed during the treatment or relapsed after the end of the treatment, and the systemic chemotherapy treatment received must be ≥two cycles. The definition of triple negative breast cancer: progesterone receptor (PR) and estrogen receptor (ER) are negative, and human epidermal growth factor receptor 2 (HER2) is negative (IHC-/+, or IHC++ with FISH/CISH-) determined by cancer tissue immunohistochemistry test.

According to the present invention, the term "combined administration" is an administration mode, and refers to administrating at least one dose of the VEGFR inhibitor and at least one dose of the poly(adenosine diphosphate-ribose) polymerase inhibitor within a certain period of time, wherein the both substances show a pharmacological effect. The period of time can be one administration cycle, preferably within 4 weeks, 3 weeks, 2 weeks, 1 week or 24 hours, and more preferably within 12 hours. The VEGFR inhibitor and the poly(adenosine diphosphate-ribose) polymerase inhibitor can be administrated simultaneously or sequentially. The period of time comprises such a treatment, wherein apatinib and the poly(adenosine diphosphate-ribose) polymerase inhibitor are administrated via the same administration route or different administration routes. The administration mode of the combination of the present invention is selected from the group consisting of simultaneous administration, co-administration after separate formulation, and sequential administration after separate formulation.

The term "effective amount" of the present invention encompasses an amount sufficient to ameliorate or prevent a symptom or sign of a medical condition. The term effective amount also refers to an amount sufficient to allow or facilitate diagnosis. An effective amount for a particular patient or veterinary subject may vary depending on factors such as the condition to be treated, the general health of the patient, the route and dose of administration, and the severity of side effects. An effective amount can be a maximal dose or an administration regimen that avoids significant side effects or toxic effects.

Overall survival (OS) refers to the date from a random day to the day of death caused by any reason. With respect to a subject that still survives at the last follow-up, the OS thereof is recorded as censored data by the last follow-up time. With respect to a subject that is lost to follow-up, the OS thereof is recorded as censored data by the last confirmed survival time before the loss of follow-up. The data censored OS is defined as the time from random grouping to censoring.

Objective response rate (ORR) refers to the proportion of patients whose tumor shrinks to a certain extent and remains for a certain period of time, including cases of CR and PR. The response evaluation criteria in solid tumors (RECIST 1.1 criteria) are employed to assess tumor objective response. The subject must have measurable tumor lesions at baseline. The efficacy assessment criteria are classified into complete response (CR), partial response (PR), stable disease (SD), and progressive disease (PD) according to the RECIST 1.1 criteria.

Disease control rate (DCR) refers to the percentage of confirmed cases of complete response, partial response and stable disease (≥eight weeks) in patients with evaluable efficacy.

Complete response (CR): all target lesions disappear, and the short diameter of all pathological lymph nodes (including target nodules and non-target nodules) must be reduced to <10 mm.

Partial response (PR): the sum of target lesion diameters is reduced by at least 30% compared to the baseline level.

Progressive disease (PD): with reference to the minimum of the sum of all measured diameters of target lesions throughout the experimental study, the sum of diameters is relatively increased by at least 20% (taking as reference the baseline value if the baseline measurement is minimum); in addition, the absolute value of the sum of diameters must be increased by at least 5 mm (the appearance of one or more new lesions is also considered to be progressive disease).

Stable disease (SD): the reduction extent of target lesions does not reach the PR level, and the increase extent does not reach the PD level either, which lie between the two, and the minimum of the sum of diameters can be used as a reference during the study.

Compound A: PARP inhibitor 4-[[3-[[2-(trifluoromethyl)-5,6,7,8-tetrahydro-[1,2,4]triazolo[1,5-α]pyrazin-7-yl]carbon yl]-4-fluorophenyl]methyl-1(2H)-phthalazinone, which can be prepared according to the method in the patent application WO2012019427A1.

Compound B: apatinib mesylate, which can be prepared according to the method in the patent application WO2010031266A1.

### DETAILED DESCRIPTION OF THE INVENTION

The following experimental solutions are provided only for the purpose of illustrating the present invention, rather than limiting the scope of the present invention. A person skilled in the art, based on the teachings in the description, can make suitable modifications or alterations to the technical solutions of the present invention without departing from the spirit and scope of the present invention.

### Example 1: Efficacy of compound A alone in treating recurrent chemotherapy-resistant ovarian cancer

In the phase I clinical trial, the dose of compound A was 120 mg/d and above. 23 patients with recurrent ovarian cancer diagnosed by histology or cytology, who had received standard treatment failure, were enrolled. These patients had been subjected to at least second-line systemic chemotherapy during the recurrence and metastasis stage of ovarian cancer. The chemotherapy regimen was a platinum-based chemotherapy regimen. These patients were platinum-based treatment-resistant, or were intolerant to the chemotherapy toxicity during the treatment.

### Administration regimen:

Compound A: the initial dose was 10 mg, 20 mg, 40 mg, 60 mg, 80 mg, 100 mg or 150 mg, administrated orally twice a day; or 120 mg or 160 mg, administrated orally once a day.

### Conclusion:

It can be seen from the clinical data of 22 evaluable cases out of 23 cases that compound A administrated alone at a daily dose of 120 mg resulted in an objective response rate (ORR) of recurrent platinum-resistant ovarian cancer of 18.2%, and a disease control rate (DCR) of 81.8%.

It can be seen from the clinical data of 13 cases that compound A administrated alone at a daily dose of 120 mg resulted in an objective response rate (ORR) of platinum-resistant ovarian cancer without BRCA mutations of 15.4%, and a disease control rate (DCR) of 84.6%.

### Example 2: Efficacy of the combination of compounds A and B of the present invention in treating recurrent chemotherapy-resistant ovarian cancer

In the phase I clinical trial, 32 patients with recurrent ovarian cancer (it should be epithelial ovarian cancer, fallopian tube cancer or primary peritoneal cancer that is high-grade serous and/or known to have BRCA1/2 dysfunctional mutation) diagnosed by histology or cytology were enrolled, who had received 2 to 4 times of platinum-based regimen treatment, the efficacy was non-PD during the last platinum-based regimen treatment, and the cancer was relapsed/progressed <6 months after the end of the treatment, or who was intolerant to the toxicity during the treatment, and the cancer was relapsed/progressed ≥6 months after the end of the penultimate platinum-based regimen treatment.

Note: toxicity intolerance: hematological toxicity of grade 4 or non-hematological toxicity of grade 3 and above occurs during the treatment.

### Administration regimen:

Compound A: the initial dose was 40 mg, 60 mg, 80 mg, 100 mg, 120 mg or 150 mg, twice a day, or 80 mg, 120 mg, 200 mg, 240 mg or 300 mg, once a day; if the first efficacy evaluation (including imaging and serological examination) was evaluated by the investigator and was considered to be not effectively relieved, and no adverse events of grade 3 or above occurred during the administration, the dose can be increased with the consent of the subject.

Compound B: the initial dose was 250 mg, administrated orally once a day.

### Data:

In the evaluable cases of drug-resistant or drug-sensitive recurrent ovarian cancer, the combination group at a daily dose of 120 mg (twice a day) resulted in an objective response rate (ORR) of platinum-resistant ovarian cancer of 100% (2/2), and a disease control rate (DCR) of 100% (2/2);
the combination group at a daily dose of 160 mg (twice a day) resulted in an objective response rate (ORR) of platinum-resistant ovarian cancer of 100% (3/3), and a disease control rate (DCR) of 100% (3/3);
the combination group at a daily dose of 160 mg (twice a day) resulted in an objective response rate (ORR) of platinum-sensitive ovarian cancer of 100% (1/1), and a disease control rate (DCR) of 100% (1/1);
the combination group at a daily dose of 200 mg (twice a day) resulted in an objective response rate (ORR) of platinum-resistant ovarian cancer of 27.27% (3/11), and a disease control rate (DCR) of 63.6% (7/11);
the combination group at a daily dose of 200 mg (twice a day) resulted in an objective response rate (ORR) of platinum-sensitive ovarian cancer of 40% (6/15), and a disease control rate (DCR) of 93.33% (14/15).

### Data analysis:

In 16 evaluable cases of platinum-resistant recurrent ovarian cancer, the combination group at a daily dose of compound A of 120 mg and above resulted in an objective response rate (ORR) of 50% (8/16), and a disease control rate (DCR) of 75% (12/16). These data are significantly better than those of the existing treatment regimen for platinum-resistant recurrent ovarian cancer (ORR, 10 to 30%).

In 16 evaluable cases of platinum-sensitive recurrent ovarian cancer, the combination group at a daily dose of compound A of 120 mg and above resulted in an objective response rate (ORR) of 43.75% (7/16), and a disease control rate (DCR) of 93.75% (15/16), which also showed an excellent clinical efficacy.

## Claims

1. Use of a poly(adenosine diphosphate-ribose) polymerase inhibitor in the preparation of a medicament for treating chemotherapy-resistant ovarian cancer or breast cancer.

2. The use according to claim 1, **characterized in that** the poly(adenosine diphosphate-ribose) polymerase inhibitor is selected from the group consisting of Olaparib, Talazoparib, Veliparib, Rucaparib, CEP-8983 and BGB-290.

3. The use according to claim 1, **characterized in that** the poly(adenosine diphosphate-ribose) polymerase inhibitor is a compound of formula (B) or a pharmaceutically acceptable salt thereof,

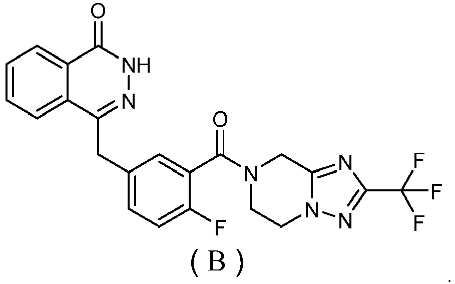

4. The use according to any one of claims 1 to 3, **characterized in that** the poly(adenosine diphosphate-ribose) polymerase inhibitor is used in combination with a VEGFR inhibitor, and the VEGFR inhibitor is preferably a VEGFR-2 inhibitor.

5. The use according to claim 4, **characterized in that** the VEGFR-2 inhibitor is a compound of formula (I) or a pharmaceutically acceptable salt thereof,

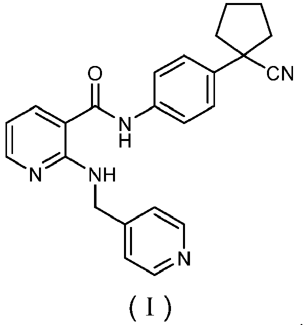

6. The use according to claim 5, **characterized in that** the pharmaceutically acceptable salt is selected from the group consisting of hydrochloride, mesylate, maleate, malate and besylate, and preferably mesylate.

7. The use according to any one of claims 1 to 6, **characterized in that** the combination has a synergistic effect.

8. The use according to any one of claims 1 to 7, **characterized in that** the chemotherapy-resistance is recurrent chemotherapy-resistance.

9. The use according to any one of claims 1 to 7, **characterized in that** the chemotherapy-resistance is platinum-based compound-resistance, and preferably recurrent platinum-based compound-resistance.

10. The use according to any one of claims 1 to 9, **characterized in that** the ovarian cancer is selected from the group consisting of epithelial ovarian cancer, high grade serous ovarian cancer and ovarian tumor.

11. The use according to any one of claims 1 to 10, **characterized in that** the breast cancer is triple negative breast cancer.

12. The use according to any one of claims 1 to 11, **characterized in that** the ratio of the daily administration dose of the VEGFR inhibitor to the daily administration dose of the poly(adenosine diphosphate-ribose) polymerase inhibitor is 0.001 to 1000, and preferably 0.83:1, 1.25:1, 1.56:1, 1.88:1.

13. The use according to any one of claims 1 to 12, **characterized in that** the administration dose of the poly(adenosine diphosphate-ribose) polymerase inhibitor is 1 to 500 mg, and preferably 80 mg, 100 mg, 120 mg, 160 mg, 200 mg or 300 mg.

14. The use according to claim 13, **characterized in that** the administration frequency of the poly(adenosine diphosphate-ribose) polymerase inhibitor is twice a day with an interval of 12 hours.

15. The use according to any one of claims 1 to 14, **characterized in that** the administration dose of the VEGFR inhibitor is 1 to 850 mg, and preferably 50 mg, 100 mg, 150 mg, 200 mg, 250 mg, 375 mg, 425 mg, 500 mg, 600 mg, 700 mg, 750 mg, 800 mg or 850 mg.

16. A pharmaceutical composition comprising the poly(adenosine diphosphate-ribose) polymerase inhibitor and the VEGFR inhibitor according to any one of claims 1 to 15, **characterized by** comprising one or more pharmaceutically acceptable excipients, diluents or vehicles.
